(19) Europäisches Patentamt
European Patent Office
Office européen des brevets

(11) Veröffentlichungsnummer: **0 192 601**
**B1**

(12) **EUROPÄISCHE PATENTSCHRIFT**

(45) Veröffentlichungstag der Patentschrift: **31.10.90**

(21) Anmeldenummer: **86810026.4**

(22) Anmeldetag: **20.01.86**

(51) Int. Cl.$^5$: **C 08 K 5/5353**, C 08 G 18/38, C 08 G 18/00, C 07 F 9/38, C 07 F 9/40

---

(54) **Flammschutzmittel für Polyurethane.**

---

(30) Priorität: **23.01.85 GB 8501704**

(43) Veröffentlichungstag der Anmeldung:
**27.08.86 Patentblatt 86/35**

(45) Bekanntmachung des Hinweises auf die Patenterteilung:
**31.10.90 Patentblatt 90/44**

(84) Benannte Vertragsstaaten:
**BE CH DE FR GB IT LI NL SE**

(56) Entgegenhaltungen:
**EP-A-0 057 668**
**EP-A-0 149 480**
**US-A-3 214 396**

(73) Patentinhaber: **CIBA-GEIGY AG**
**Klybeckstrasse 141**
**CH-4002 Basel (CH)**

(72) Erfinder: **Richardson, Norman**
**21, Grey Street**
**Middleton Manchester M24 3UF (GB)**
Erfinder: **Clubley, Brian George**
**7 Green Villa Park**
**Wilmslow Cheshire SK9 6EJ (GB)**
Erfinder: **Dellar, Richard John, Dr.**
**17 Cleveland Road Hale**
**Altrincham Cheshire WA15 8AY (GB)**

**EP 0 192 601 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Vorliegende Erfindung betrifft die Verwendung organischer Phosphonsäuresalze zur Erhöhung der Flammwidrigkeit von Polyurethanen und Polyisocyanuraten sowie neue organische Phosphonsäuresalze.

Die Flammwidrigkeit von Polyurethanen und Polyisocyanuraten wird gewöhnlich durch Zusatz einer phosphorhaltigen Verbindung, einer halogenhaltigen Verbindung oder Gemischen von diesen erhöht. Eine weithin verwendete phosphorhaltige Verbindung ist Dimethyl-methylphosphonat (DMMP) — siehe z.B. GB—A—1 094 717, 999 588 und 919 067 sowie US—A—4 165 411. Bei der Verwendung von DMMP treten jedoch gewisse Probleme auf. Erstens ist dies eine verhältnismässig flüchtige Verbindung (Siedepunkt 181°C), was bedeutet, dass Material unter gewissen Umständen durch Verflüchtigung verlorengehen kann. Zweitens wird DMMP gewöhnlich mit zur Herstellung von Polyurethanen und Polyisocyanuraten verwendeten Polyolen zusammen mit Katalysatoren, Treibmitteln und weiteren Bestandteilen vor dem Zusatz des Isocyanats formuliert. Die Formulierung zeigt eine Tendenz zu einer gewissen Instabilität, wenn sie vor der Verwendung längere Zeit gelagert wird. Die Viskosität solcher Formulierungen kann beim Stehen ansteigen, und veränderliche Schäumeigenschaften sind beim Vermischen mit dem Isocyanatreaktionspartner zu beobachten.

Einige Salze von u.a. DMMP mit Aminoverbindungen sind bekannt und im EP—A—57 668 und US—A—4 308 197 beschrieben. Die Verwendung solcher Salze als Flammhemmzusätze für Polyole oder Polyurethane wird jedoch nicht erwähnt.

Es ist Aufgabe der vorliegenden Erfindung, phosphorhaltige Flammschutzmittel mit geringer Flüchtigkeit zur Verfügung zu stellen, welche Polyolformulierungen ergeben, die bei der Lagerung stabil sind.

Gegenstand vorliegender Erfindung sind demnach Polyurethane oder Polyisocyanurate mit einer darin eingebauten, flammhemmenden Menge eines Salzes, gebildet durch Umsetzung von Dimethyl-methylphosphonat, Monomethyl-methylphosphonat oder Methylphosphonsäure mit einer Verbindung der allgemeinen Formel (I)

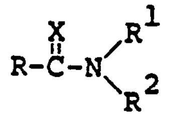

I

worin X für O, S oder NH, $R^1$ für H, Alkyl mit 1 bis 4 Kohlenstoffatomen, Alkenyl mit bis zu 4 Kohlenstoffatomen, CN, $CONH_2$ oder $NH_2$ und $R^2$ für H, Alkyl mit 1 bis 4 Kohlenstoffatomen oder Alkenyl mit bis zu 4 Kohlenstoffatomen stehen, oder $R^1$ und $R^2$ zusammen mit dem Stickstoffatom, an das sie gebunden sind, einen heterocyclischen Ring mit bis zu 6 Kohlenstoffatomen bilden, der gegenbenenfalls ein weiteres Heteroatom enthalten kann, und R für H, eine Alkylgruppe mit 1 bis 8 Kohlenstoffatomen, eine Arylgruppe mit 6 bis 10 Kohlenstoffatomen, eine Cycloalkylgruppe mit 5 bis 12 Kohlenstoffatomen oder eine heterocyclische Gruppe mit bis zu 9 Ringkolhenstoffatomen steht oder zusammen mit $R^1$ eine Alkylenkette mit 3 bis 10 Kohlenstoffatomen bildet, oder R für eine Gruppe $NHR^3$ steht, worin $R^3$ H, Alkyl mit 1 bis 4 Kohlenstoffatomen, Alkenyl mit bis zu 4 Kohlenstoffatomen, CN, $CONH_2$ oder $NH_2$ bedeutet oder zusammen mit $R^1$ eine Alkylenkette mit 2 oder 3 Kohlenstoffatomen bildet, oder R für eine Gruppe

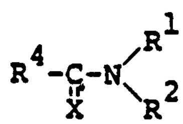

steht, worin $R^1$, $R^2$ und X die obigen Bedeutungen haben und $R^4$ eine direkte Bindung, eine Alkylengruppe mit bis zu 8 Kohlenstoffatomen oder eine Arylengruppe mit 6 bis 10 Kohlenstoffatomen darstellt.

Verschiedene Verbindungen der Formel (I) sind verwendbar. Eine geeignete und bevorzugte Klasse von Verbindungen entspricht der allgemeinen Formel (II)

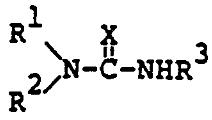

II

worin $R^1$, $R^2$, $R^3$ und X die obigen Bedeutungen haben.

Eine weitere geeignete Klasse von Verbindungen entspricht der allgemeinen Formel (III)

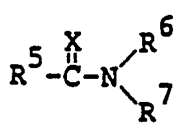

III

worin $R^5$ für H, Alkyl mit 1 bis 8 Kohlenstoffatomen oder eine Aryl-, Cycloalkyl- oder heterocyclische Gruppe steht, $R^6$ und $R^7$ gleich oder verschieden sind und H oder eine Alkylgruppe mit 1 bis 4 Kohlenstoffatomen

bedeuten oder $R^6$ und $R^7$ zusammen mit dem Stickstoffatom, an das sie gebunden sind, einen heterocyclischen Ring mit bis zu 6 Kohlenstoffatomen bilden, der gegebenenfalls ein weiteres Heteroatom enthalten kann, und X die oben angegebene Bedeutung hat.

Eine weitere geeignete Klasse von Verbindungen entspricht der allgemeinen Formel

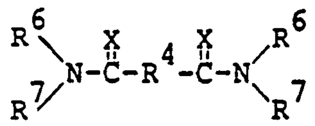

IV

worin $R^4$, $R^6$ und $R^7$ die oben angegebenen Bedeutungen haben.

Eine weitere geeignete Klasse von Verbindungen entspricht der allgemeinen Formel (V)

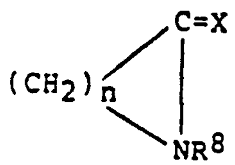

V

worin $R^8$ für H oder Alkyl mit 1 bis 4 Kohlenstoffatomen und n für eine ganze Zahl von 3 bis 10 stehen und X die obige Bedeutung hat.

R oder $R^5$ als $C_1$—$C_8$-Alkylgruppe können beispielsweise Methyl, Aethyl, Isopropyl, n-Butyl, sek.-Butyl, Hexyl oder Octyl sein.

$R^1$, $R^2$, $R^3$, $R^6$, $R^7$ oder $R^8$ als $C_1$—$C_4$-Alkylgruppen können beispielsweise Methyl, Aethyl, Isopropyl, n-Butyl oder sek.-Butyl sein.

$R^1$, $R^2$ oder $R^3$ als Alkenyl mit bis zu 4 Kohlenstoffatomen können beispielsweise Allyl, Propenyl oder Butenyl sein.

$R^1$ und $R^2$ oder $R^6$ und $R^7$, wenn sie einen heterocyclischen Ring bilden, können beispielsweise einen Pyridin-, Morpholin-, Piperidin- oder Piperazinring darstellen.

R als heterocyclischer Ring mit bis zu 9 Kohlenstoffatomen kann beispielsweise einen Pyridin-, Morpholin-, Piperidin-, Indol- oder Chinolinring darstellen.

R als Arylring mit 6—10 Kohlenstoffatomen kann beispielsweise einen Benzol- oder Naphthalinring darstellen.

R als Cycloalkylring mit 5—12 Kohlenstoffatomen kann beispielsweise einen Cyclopentyl-, Cyclohexyl-, Cyclooctyl- oder Cyclododecylring darstellen.

$R^5$ als Aryl-, Cycloalkyl- oder heterocyclische Gruppe kann einen wie oben für R beschriebenen Rest darstellen.

Wenn R und $R^1$ zusammen eine Alkylenkette mit 3 bis 10 Kohlenstoffatomen bilden, so kann diese beispielsweise Propylen, Butylen, Hexylen oder Dekamethylen darstellen.

$R^4$ als Alkylengruppe mit bis zu 8 Kohlenstoffatomen kann beispielsweise Methylen, Aethylen, Propylen, Hexylen oder Octylen sein.

$R^4$ als Arylengruppe mit 6 bis 10 Kohlenstoffatomen kann beispielsweise Phenylen oder Naphthylen sein.

Die erfindungsgemäss verwendeten Salze sind herstellbar durch Vermischen von Dimethyl-methylphosphonat, Monomethyl-methylphosphonat oder Methylphosphonsäure mit einer Verbindung der oben definierten allgemeinen Formel (I), gegebenenfalls in einem wässrigen oder organischen Lösungsmittel und gegebenenfalls in einer Inertgasatmosphäre sowie nötigenfalls unter Erhitzen zwecks Salzbildung.

Dabei wird eine solche Menge Dimethyl-methylphosphonat, Monomethyl-methylphosphonat oder Methylphosphonsäure eingesetzt, dass sie mit einem oder mehreren salzbildenden Stickstoffatomen in der Verbindung der Formel (I) wahlweise zu einem Monosalz, Disalz oder höheren Salz reagiert. Normalerweise wird ein Monosalz oder Disalz hergestellt, was ein oder zwei Mol Phosphonsäure oder -ester pro Mol Verbindung der Formel (I) erfordert.

Bei Verwendung von Methylphosphonsäure oder deren Monomethylester erfolgt die Salzbildung schnell, und milde Bedingungen sind anwendbar. Man kann einfach die Säure und die Verbindung der Formel (I) bei Raumtemperatur zur Salzbildung vermischen. Ist die Verbindung der Formel (I) eine viskose Flüssigkeit oder ein Feststoff, so kann man das Gemisch erhitzen, z.B. bis auf 100°C, um für eine wirksame Umsetzung zu sorgen. Gewünschtenfalls kann man ein wässriges oder organisches Lösungsmittel verwenden, das nach beendeter Reaktion entfernt wird.

Bei Vewendung von DMMP sind schärfere Reaktionsbedingungen erforderlich, um Salzbildung zu erzwingen. Man kann das Reaktionsgemisch bis auf 180°C erhitzen, gegebenenfalls unter einem inerten Gas wie Stickstoff. Mehrere Stunden langes Erhitzen auf 100°C bis 180°C kann notwendig sein. Gewünschtenfalls kann man die Reaktion in einem wässrigen oder organischen Lösungsmittel, beispielsweise einem Kohlenwasserstofflösungsmittel wie Toluol oder Xylol, durchführen.

Methylphosphonsäure und ihre Ester sowie die Verbindungen der Formel (I) sind sämtlich bekannte Verbindungen und lassen sich auf verschiedenen Wegen herstellen.

Die erfindungsgemäss verwendeten Salze sind neue Verbindungen, mit Ausnahme von denen, die aus Methylphosphonsäure und Dicyandiamid oder Methylphosphonsäure und Guanidin gebildet werden. Diese neuen Verbindungen sind ebenfalls Gegenstand der vorliegenden Erfindung.

Beispiele für zur Salzbildung verwendbare Verbindungen der Formel (I) sind unter anderem Harnstoff, Dicyandiamid, Guanidin, Aminoguanidin, Thioharnstoff, N-Methylharnstoff, N-Allylharnstoff, N,N-Diallylharnstoff, N,N'-Dimethylharnstoff, Aethylenharnstoff, Biuret, (Thio)acetamid, (Thio)propionamid, (Thio)butyramid, (Thio)octanamid, Malonamid, Mono- oder Dithiomalonamid, Succinamid, Mono- oder Dithiosuccinamid, Sebacamid, Pyrrolidon, Piperidinon, (Thio)caprolactam und (Thio)dodecalactam.

Die erfindungsgemäss verwendeten Slze können für sich oder zusammen mit anderen Flammschutzmitteln wie den unten angegebenen eingesetzt werden, um hartgen oder weichen Polyurethanen oder Polyisocyanuraten Flammwidrigkeit zu verleihen. Polyurethane und Polyisocyanurate werden durch Umsetzung eines Toluols mit einem Polyisocyanat in Gegenwart eines Treibmittels, falls ein Schaum erwünscht ist, und eines Katalysators hergestellt. Die Menge Polyisocyanat wird zur Erzeugung des gewüschten Produkts variiert. Vorliegende Erfindung ist auf den ganzen Bereich von Polymeren mit einem Isocyanatindex von 1 bis 6, vorzugsweise von 1 bis 4,5, anwendbar.

Die erfindungsgemäss verwendeten Salze kann man in das Polyurethan oder Polyisocyanurat einbauen, indem man sie dem zur Herstellung des Polyurethans oder Polyisocyanurats verwendeten Reaktionsgemisch zusetzt. Ferner kann man sie dem hydroxylgruppenhaltigen Reaktionspartner (Polyester- oder Polyäther-polyol) vor dessen Reaktion mit dem Polyisocyanat zusetzen. Die Viskosität des Polyol/Salzgemisches ändert sich nicht erheblich während der Lagerung bei Raumtemperatur, und man erreicht konstante Schäumbedingungen, die sich mit der Lagerperiode nicht ändern.

Die in das Polyurethan oder Polyisocyanurat einzubauende Menge flammhemmendes Salz hängt vom Grad der erforderlichen Flammwidrigkeit ab. Die Menge an flammhemmendem Salz kann typischerweise 1 bis 100 Teile, vorzugsweise 3 bis 50 Gewichtsteile auf hundert Gewichtsteile Polyol betragen.

Die Salze können mit anderen flammhemmenden Verbindungen vermischt werden. Dabei handelt es sich beispielsweise um halogenhaltige Verbindungen wie aliphatische und aromatische Bromverbindungen, oxyalkylierte Phosphatester, Chloralkylphosphate, -phosphonate oder Triarylphosphate. Geeignete Beispiele sind Pentabromdiphenyläther, Dibromkresylglycidyläther, Tetrabrombisphenol-A, Dibromneopentylglykol, ein durch Umsetzung von Tetrabromphthalsäureanhydrid mit Aethylenoxyd und/oder Propylenoxyd hergestelltes Diol, Tris-(chloräthyl)-phosphat, Tris-(monochlorpropyl)-phosphat, Diäthyl-bis-(hydroxyäthyl)-aminomethylphosphonat sowie isopropylierte oder t-butylierte Phenylphosphatgemische wie im britischen Patent 1 146 173 beschrieben, Trikresylphosphat, Trixylylphosphat und Kresyldiphenylphosphat.

Die erfindungsgemässen Salze sind ferner einsetzbar als Gemisch mit Phosphonsäureaminsalzen der allgemeinen Formel (VI) und/oder (VII)

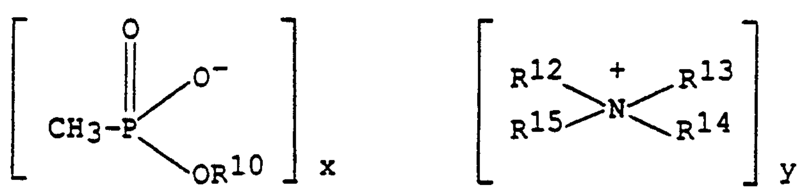

(VI)

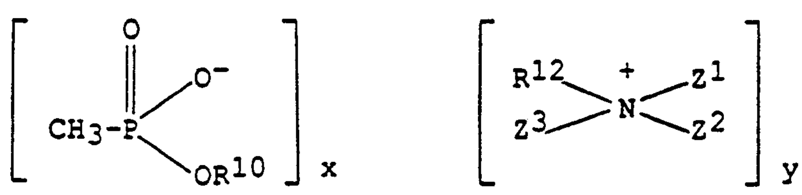

(VII)

worin x und y solche ganze Zahlen sind, dass die Anzahl negativer und positiver Ladungen gleich ist, $R^{10}$ und $R^{12}$ für Wasserstoff oder Methyl und $R^{13}$ für eine durch 1—3 gegebenenfalls durch eine Oxyalkylenkette substituierte Hydroxylgruppen substituierte $C_2$—$C_4$-Alkyl-gruppe stehen, wobei sich jeweils nicht mehr als eine Hydroxylgruppe an einem Kohlenstoffatomen befindet, $R^{14}$ und $R^{15}$ gleich oder verschieden sein und dieselbe Bedeutung wie $R^{13}$ haben oder für Wasserstoff, eine $C_1$—$C_4$-Alkylgruppe oder eine gegebenenfalls durch eine Alkylgruppe mit 1—12 Kohlenstoffatomen, eine gegebenenfalls durch eine Oxyalkylenkette substituierte Hydroxylgruppe und/oder 1—3 Halogenatome am aromatischen Ring substituierte Phenyl- oder Benzylgruppe stehen können oder $R^{15}$ eine Gruppe der Formel (VIII)

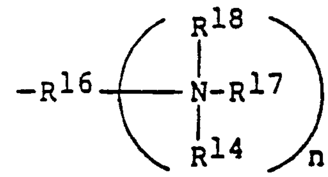

VIII

darstellt, worin $R^{16}$ für eine Alkylengruppe mit 2—4 Kohlenstoffatomen, eine Phenylengruppe, eine Xylylengruppe, eine Diphenylmethangruppe oder eine Gruppe der Formel

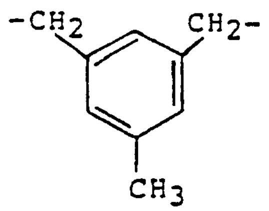

oder

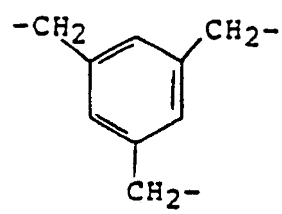

steht, wobei aromatische Ringe in einer Gruppe $R^{16}$ gegebenenfalls durch eine Alkylgruppe mit 1—12 Kohlenstoffatomen, eine gegebenenfalls durch eine Oxyalkylenkette substituierte Hydroxylgruppe und/oder 1—3 Halogenatome substituiert sein können, $R^{17}$ für Wasserstoff oder Methyl, wobei das Stickstoffatom dann eine positive Ladung trägt, steht oder fehlt, $R^{18}$ für Wasserstoff oder eine wie für $R^{13}$ angegebene Gruppe steht und n 1 oder 2 ist, oder $R^{14}$ und $R^{15}$ gegebenenfalls mit dem Stickstoff unter Bildung eines gegebenenfalls ein Sauerstoffatom enthaltenden, 5- oder 6-gliedrigen heterocyclischen Rings verknüpft sind, sowie $Z^1$, $Z^2$ und $Z^3$ gleich oder verschieden sind und Wasserstoff, eine geradkettige oder verzweigte $C_1$—$C_{12}$-Alkylgruppe, eine geradkettige oder verzweigte $C_3$—$C_{12}$-Alkinylgruppe, eine $C_4$—$C_{12}$-Cycloalkylgruppe, eine gegebenenfalls durch eine geradkettige oder verzweigte $C_1$—$C_4$-Alkylgruppe, eine $C_1$—$C_4$-Alkoxygruppe, Amino, Methylamino, Halogen oder Nitro substituierte Phenyl- oder Naphthylgruppe oder eine $C_7$-$C_{12}$-Aralkylgruppe bedeuten, oder $Z^2$ und $Z^3$ zusammen mit dem Stickstoffatom, an das sie gebunden sind, ein gesättigtes oder ungesättigtes, 3- bis 7-gliedriges Ringsystem bilden, das gegebenenfalls ein weiteres Heteroatom enthalten kann und gegebenenfalls durch eine geradkettige oder verzweigte $C_1$—$C_4$-Alkylgruppe, eine $C_1$—$C_4$-Alkoxygruppe, Amino, Methylamino, eine $C_1$—$C_4$-Aminoalkylgruppe, Halogen oder Nitro substituiert ist, oder $Z^1$, $Z^2$ oder $Z^3$ zusammen mit dem Stickstoffatom, an das sie gebunden sind, einen gegebenenfalls ein weiteres Heteroatom enthaltenden, 8- bis 12-gliedrigen bicyclischen Ring bilden, oder $Z^1$ eine Gruppe der Formel

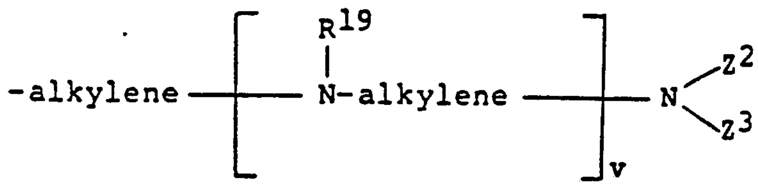

darstellt, worin Alkylen eine Gruppe mit 2 bis 12 Kohlenstoffatomen, v 0 oder eine ganze Zahl von 1 bis 5 und $R^{19}$ Wasserstoff oder eine geradkettige oder verzweigte $C_1$—$C_{16}$-Alkylgruppe bedeuten und $Z^2$ und $Z^3$ die obige Bedeutung haben, oder $Z^1$ eine Gruppe der Formel

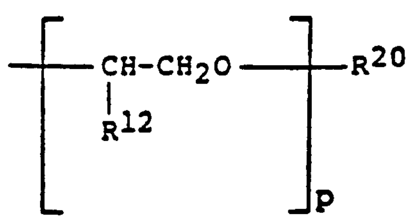

oder

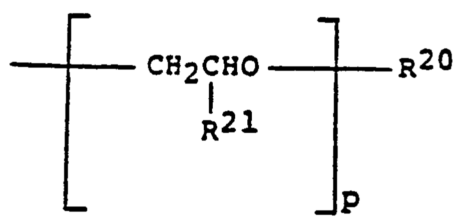

darstellt, worin $R^{12}$ die obige Bedeutung hat und $R^{20}$ für, eine $C_1$—$C_{12}$-Alkylgruppe und p für eine ganze Zahl von 1—10, vorzugsweise 1—4, stehen oder $Z^3$ eine Gruppe der Formel (VIIIa)

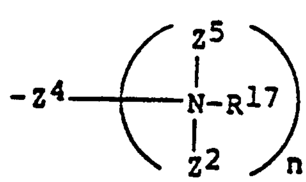

VIIIa

darstellt, worin $Z^4$ für eine Alkylengruppe mit 2—12 Kohlenstoffatomen, eine Phenylengruppe, eine Xylylengruppe, eine Diphenylmethangruppe oder eine Gruppe der Formel

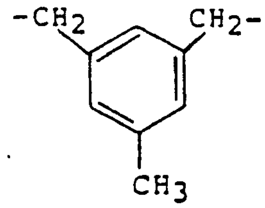

oder

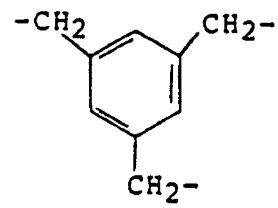

steht, wobei aromatische Ringe in einer Gruppe $Z^4$ gegebenenfalls durch eine Alkylgruppe mit 1—12 Kohlenstoffatomen, Hydroxyl und/oder 1—3 Halogenatome substituiert sind, und $R^{17}$ für Wasserstoff oder Methyl, wobei das Stickstoffatom dann eine positive Ladung trägt, steht oder fehlt, $Z^5$ Wasserstoff oder eine wie für $Z^1$ angegebene Gruppe bedeutet und n 1 oder 2 ist, oder $Z^2$ und $Z^5$ gegebenenfalls mit dem Stickstoff unter Bildung eines gegebenenfalls ein weiteres Heteroatom enthaltenden 3- bis 7-gliedrigen heterocyclischen Rings verknüpft sind.

Die Salze der Formeln (VI) und (VII) sind herstellbar durch Vermischen von Methylphosphonsäure oder -ester der Formel

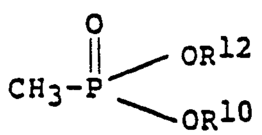

IX

worin $R^{12}$ die obige Bedeutung hat und $R^{10}$ für Wasserstoff oder Methyl steht, mit einem Amin der Formel X und/oder XI

X 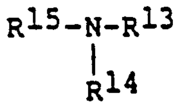 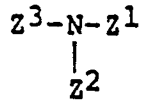 XI

worin $R^{13}$, $R^{14}$, $R^{15}$, $Z^1$, $Z^2$ und $Z^3$ die obigen Bedeutungen haben, gegebenenfalls in einem wässrigen oder organischen Lösungsmittel und gegebenenfalls in einer Inertgasatmosphäre sowie nötigenfalls unter Erhitzen zwecks Salzbildung.

Dabei wird eine solche Menge Methylphosphonsäure oder -ester der Formel (IX) eingesetzt, dass sie mit einer oder mehreren der Amingruppen im Amin der Formel (X) oder (XI) wahlweise zu einem Monosalz, Disalz oder höheren Salz reagiert.

Das Verhältnis des Salzes zu anderen flammhemmenden Verbindungen kann 5:95 bis 95:5 betragen.

Die Zusammensetzungen können ferner Methylphosphonsäure und deren Monomethyl- oder Dimethylester enthalten, die nicht mit der vorhandenen Verbindung der Formel (I) umgesetzt sind, doch enthält die Formulierung vorzugsweise keine sauren Zusatzstoffe.

Jegliche aus dem Stand der Technik bekannte Isocyanate und Polyole sind bei der Herstellung der Polyurethane oder Polyisocyanurate verwendbar.

Das Isocyanat ist gewöhnlich eine Flüssigkeit, wie Toluoldiisocyanat, Methylendiphenyldiisocyanat, polymeres Methylendiphenyldiisocyanat, hydriertes Methylendiphenyldiisocyanat, Hexamethylendiisocyanat, Isophorondiisocyanat sowie irgendwelche Polyisocyanatpräpolymere mit zwei oder mehr nicht umgesetzten Isocyanatresten und dergleichen. Ueblicherweise enthält das erfindungsgemäss verwendete Toluoldiisocyanat die Isomeren 2,4- und 2,6-Toluoldiisocyanat. Die Konzentration dieser Isomeren ist unerheblich.

Das Polyol für Hartschaum kann eine polyfunktionelle aktive Wasserstoffverbindung sein, die von der Reaktion einer Polyhydroxylverbindung wie Glycerin, Saccharose, Sorbit, Trimethylolpropan, Pentaerythrit, Triäthanolamin oder einem Amin wie Aethylendiamin, einem polyaromatischen Amin oder einer aromatischen Mannichbase mit Propylenoxyd und/oder Aethylenoxyd hergeleitet ist.

Zur Herstellung von Polyurethanweichschäumen sind die Polyole allgemein Polyätherpolyole wie Polyoxyäthylen/Oxypropylendiole, Polyoxyäthylen/Oxyproylentriole, Rizinusöl und Methylglucosidpolyätherpolyole mit Durchschnittsmolekulargewichten im Bereich von ungefähr 250—6500. Weiterhin sind anstelle der Polyätherpolyole auch Polyesterpolyole verwendbar, wie die Reaktionsprodukte einer aliphatischen difunktionellen Carbonsäure, z.B. Adiopinsäure oder Sebacinsäure, mit einer di- oder trifunktionellen Hydroxylverbindung, z.B. Aethylenglykol, Diäthylenglykol, Propylenglykol, 1,4-Butylenglykol und Butantriol.

Zusätzlich können Poylole wie Glycerin, Hexantriol, Butantriol, Trimethylolpropan, Trimethyloläthan und Pentaerythrit bei der Polymerisationsreaktion mit dem Polyol mitverwendet werden, um ein erwünschtes, stöchiometrisch ausgeglichenes Isocyanat/Hydroxylverhältnis aufrechtzuerhalten.

Bei der Herstellung geschäumter Polyurethane und Polyisocyanurate kann man irgendwelche herkömmliche basische Katalysatoren einsetzen, wie zum Beispiel Natriumhydroxyd, Natriumacetat, tertiäre Amine oder Stoffe, die tertiäre Amine freisetzen, wie Trimethylamin, Triäthylendiamin, N-Methylmorpholin, N,N-Dimethylcyclohexylamin und N,N-Dimethylaminoäthanol. Ferner sind Metallverbindungen einsetzbar, wie Kohlenwasserstoff-zinnalkylcarboxylate, Dibutylzinndiacetat, Dibutylzinndioctoat, Dibutylzinndilaurat und Zinn(II)-octoat sowie weitere, zur Förderung der Trimerisierung des Isocyanats bestimmte Verbindungen wie 2,4,6-Tris-(N,N-dimethylaminomethyl)-phenol, 1,3,5-Tris-(N,N-dimethyl-3-aminopropyl)-s-hexahydrotriazin, Kaliumoctoat, Kaliumacetat und Katalysatoren, wie sich unter den Markennamen DABCO®, TMR® und POLYCAT 43® im Handel befinden.

Die oben angeführten Katalysatoren können wie gewünscht durch zahlreiche andere Katalysatoren ersetzt werden. Die Katalysatoreinsatzmenge kann im Bereich von etwa 0,05 Gew.-% bis zu etwa 5 Gew.-% oder höher liegen, bezogen auf das Gesamtgewicht des eingesetzten Polyols bzw. der Polyole. Gemische der obigen und/oder weiterer Katalysatoren sind ebenfalls verwendbar.

Um dem vermengten Polyol/Polyisocyanatgemisch eine Schaum- oder Zellstruktur zu verleihen, muss ein geeignetes Treibmittel oder Treibmittelsystem zugesetzt oder an Ort und Stelle erzeugt werden. Zu geeigneten Treibmitteln gehören flüssige, aber verhältnismässig flüchtige halogenierte Kohlenwasserstoffe wie Dichlordifluormethan, Trichlorfluormethan und Methylenchlorid. Diese gibt man als Flüssigkeiten in Mengen von etwa 5 Gew.-% bis etwa 50 Gew.-% auf Polyol zu einer oder mehreren Komponenten des Polyol/Polyisocyanatgemischs; diese werden im flüssigen Gemisch weitgehend unter Zellbildung verflüchtigt. Danach härtet das Gemisch zu seiner fertigen zelligen Gestalt aus.

Obwohl die halogenierten Kohlenwasserstoffe als Treibmittel besonders erwünscht sind, wenn ausserordentliche Isoliereigenschaften erforderlich sind, kann man auch andere Treibmittel, wie durch Zusatz von Wasser zum Polyol oder gleichzeitig mit der Zugabe des Polyisocyanats erzeugtes Kohlendioxyd, insbesondere für offenzellige Weichschäume einsetzen.

Es sei auch bemerkt, dass das Schäumen auch durch kombinierte Anwendung eines Treibmittels und Wasserzusatz zum Polyol bewirkt werden kann.

Um eine verhältnismässig gleichförmige Verteilung der verschiedenen Komponenten des flüssigen Systems zu erhalten und die erwünschte Blasenbildung zu erreichen, kann man einen Emulgator und/oder ein Tensid in das Gemisch einarbeiten. Diese Stoffe zeigen eine physikalische Wirkung und sind nicht immer notwendig, insbesondere wenn dichtere Schäume herzustellen sind. Dabei kann man aus den Hunderten von herkömmlichen Tensiden irgendeines in Mengen bis zu 4% bezogen auf das Gewicht des verwendeten Polyols einsetzen. Als Tenside sind Polydimethylsiloxan und Polydimethylsiloxan/Polyalkylencopolymere und dergleichen, wie aus dem Stand der Technik bekannt, geeignet.

Vorliegende Erfindung umfasst ferner den Einsatz weiterer Stoffe in den erfindungsgemässen Zusammensetzungen, wo ein bestimmtes Endergebnis zu erzielen ist. Zu solchen Stoffen zählen ohne Einschränkung Haftvermittler, Antioxydantien, Antistatika, Bakteriostatika, Farbstoffe, Wärmestabilisatoren, Lichtschutzmittel, Pigmente, Weichmacher, Konservierungsmittel, Ultraviolettschutzmittel und Füllstoffe, wie in Modern Plastics Encyclopedia [Moderne Kunststoffenzyklopädie], Band 58, Nr. 10A, S. 170—187 beschrieben.

Die Erfindung wird in den nachfolgenden Beispielen erläutert, wobei "Teile" Gewichtsteile bedeuten.

Beispiel 1

Man erhitzt 42,0 g (0,5 Mol) Dicyandiamid und 62 g (0, 5 Mol) Dimethy-methylphosphonat in einer Stickstoffatmosphäre auf 140°C und hält diese Temperatur 4 Stunden lang aufrecht. Dann kühlt man auf 50°C und rüstet den Apparat auf Vakuumdestillaton um. Nicht umgesetztes Dimethylmethylphosphonat wird bei einem Druck von 15,6 mbar bis zu einer Innentemperatur von 140°C entfernt.

Dabei erhält man 74,5 g bernsteinfarbenes Harz.

Analyse zeigt, dass das Produkt im wesentlichen aus

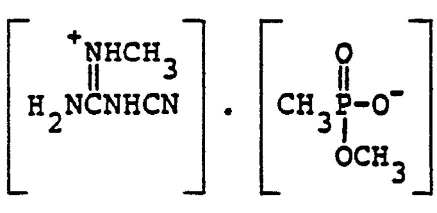

verunreinigt mit etwa 16% nicht umgesetztem Dicyandiamid besteht.

Beispiel 2

Man erhitzt 600 g (10 Mol) Harnstoff und 1240 g (10 Mol) Dimethyl-methylphosphonat 14 Stunden lang in einer Stickstoffatmosphäre auf 140°C. Man kühlt auf 50°C und rüstet dann den Apparat für Vakuumdestillation um. Nicht umgesetzte Ausgangsstoffe werden durch Destillation bei einem Druck von 15,6 mbar bis zu einer Innentemperatur von 140°C entfernt.

Dabei erhält man 1654,8 g farbloses viskoses flüssiges Amidsalz mit der folgenden Elementaranalyse:

C, 25,90%; H, 7.13%; N, 15,47% und P, 16,62%. Berechnet für $C_4H_{13}N_2O_4P$ C, 26,09%; H, 7,07%; N, 15,22% und P, 16,85%.

Beispiel 3 (nicht erfindungsgemäß)

Man gibt eine Lösung von 96 g (1 Mol) Methylphosphonsäure in 100 ml Wasser im Verlauf von 1 Stunde bei 25°C zu einer Lösung von 90 g (0,5 Mol) Guanidincarbonat in 100 ml Wasser. Dabei entwickelt sich Kohlendioxyd aus der Lösung. Man rührt noch 2 Stunden lang bei 25°C und dampft dann zur Trockne ein. Dabei erhält man 150,6 g farblosen kristallinen Feststoff mit der Analyse C, 15,03%; H, 6,73%; N, 27,05% und P, 19,79%. Berechnet für $C_2H_{10}N_3O_3P$ C, 15,48%; H, 6,45%; N, 27,10% und P, 20,0%.

Beispiel 4

Man gibt eine Lösung von 96 g (1 Mol) Methylphosphonsäure in 100 ml Wasser im Verlauf von 1 Stunde zu einer Dispersion von 60 g (1 Mol) Harnstoff in 60 g Wasser. Nach 15 Minuten bildet sich eine klare Lösung. Diese wird noch eine halbe Stunde lang gerührt und dann zur Trockne eingedampft. Dabei erhält man 155 g farblose viskose Flüssigkeit mit der Analyse C, 15,43%; H, 5,65%; N, 17,89% und P, 19,91%. Berechnet für $C_2H_9N_2O_4P$ C, 15,38%; H, 5,77%; N, 17.95% und P, 19,87%.

Beispiel 5 (nicht erfindungsgemäß)

Man gibt 84 g (1 Mol) Dicyandiamid portionsweise im Verlauf von 25 Minuten bei 90°C zu einer Lösung von 96 g (1 Mol) Methylphosphonsäure in 180 g Wasser. Man erhitzt die Lösung noch 45 Minuten lang auf 90°C und dampft dann zur Trockne ein. Dabei erhält man 188,7 g farblosen kristallinen Stoff, der durch Umkristallisieren aus 1,5 Litern Aethylalkohol gereinigt wird. Dabei erhält man 63,4 g Substanz vom Schmelzpunkt 163—4°C und der folgenden Analyse: C, 18,53%; H, 5,40%; N, 28,43% und P, 15,90%. Dies entspricht $C_3H_{11}N_4O_4P$. Das Infrarotspektrum zeigt keinen Nitrilpeak (−C=N). Während der Herstellung hydrolysiert die Nitrilgruppe zu Amid, wobei die Verbindung

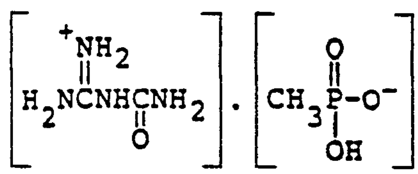

entsteht.

Beispiel 6

Man erhitzt 135 g (0,96 Mol) N,N-Diallylharnstoff in einer Stickstoffatmosphäre auf 100°C, wobei man gleichzeitig 119,6 g (0,96 Mol) Dimethy-methylphosphonat im Verlauf von 2 Stunden dazutropft. Man erhitzt noch 5 1/2 Stunden lang auf 100°C und kühlt dann auf Raumtemperatur. Die Apparatur wird zur Vakuumdestillation umgerüstet, und nicht umgesetzte Ausgangsstoffe werden durch Erhitzen auf 100°C bei 19,5 mbar Druck entfernt. Dabei erhält man 253 g schwachgelbe Flüssigkeit mit der Elementaranalyse C, 45,03%; H, 8,24%; N, 10,37% und P, 11,96%. Berechnet für $C_{10}H_{21}N_2O_4P$ C, 45,45%; H, 7,95%; N, 10,60% und P, 11,74%.

Beispiel 7

Man vermischt 100 g (1 Mol) Allylharnstoff und 124 g (1 Mol) Dimethyl-methylphosphonat und erhitzt in einer Stickstoffatmosphäre insgesamt 22 1/2 Stunden lang auf 140°C. Man kühlt auf 25°C und rüstet die Apparatur für Vakuumdestillation um. Nicht umgesetzte Ausgangsstoffe werden durch Erhitzen auf 140°C bei 19,5 mbar Druck entfernt. Dabei erhält man 193,1 g schwachgelbe Flüssigkeit mit einem Phosphorgehalt von 13,88%. Berechnet für $C_7H_{17}N_2O_4P$, P = 13,84%.

Beispiel 8

Man bringt 74 g (1 Mol) N-Methylharnstoff und 124 g (1 Mol) Dimethyl-methylphosphonat in einer Stickstoffatmosphäre bei 140°C für insgesamt 17 1/2 Stunden zur Reaktion. Nach Entfernung nicht umgesetzter Ausgangsstoffe durch Vakuumabstreifung bei 15,6 mbar Druck erhält man dabei 172,3 g farblose Flüssigkeit mit einem Phosphorgehalt von 15,92%. Berechnet für $C_5H_{15}N_2O_4P$, P = 15,65%.

Beispiel 9

Man gibt 103 g (1 Mol) N-(2-Hydroxyäthyl)-acetamid im Verlauf von 2 Stunden bei 140°C zu 124 g (1 Mol) Dimethyl-methylphosphonat. Man erhitzt noch 21 1/2 Stunden lang auf 140°C. Nach Entfernung nicht umgesetzter Ausgangsstoffe durch Vakuumabstreifung bei 19,5 mbar Druck erhält man dabei 187 g schwachgelbe Flüssigkeit mit einem Phosphorgehalt von 13,86%. Berechnet für $C_7H_{18}NO_5P$, P = 13,66%.

Beispiel 10

Man erhitzt 103 g (1 Mol) Biuret und 124 g Dimethylmethylhosphonat in einer Stickstoffatmosphäre insgesamt 11 1/2 Stunden lang auf 140°C. Nach Entfernung nicht umgesetzter Ausgangsstoffe im Vakuum bei 19,5 mbar Druck erhält man dabei 186 g weissliche viskose Flüssigkeit mit einem Phosphorgehalt von 14,65%, berechnet = 13,66%, die 9,0% nicht umgesetzten Biuret enthält.

Biespiel 11

In einem mit Rührer, Thermometer und Fraktionierkolonne ausgerüsteten Vierhalskolben erhitzt man 59 g (1 Mol) Acetamid und 124 g (1 Mol) Dimethyl-methylphosphonat insgesamt 24 Stunden lang auf 140°C. Das Methylacetatnebenprodukt wird in der Fraktionierkolonne abgetrennt und über einen Wasserkühler in einer Vorlage gesammelt.

Nach Abkühlen auf 25°C und 24 Stunden Stehen isoliert man 22,8 g schwachgelbe Kristalle durch Filtrieren. Dieser kristalline Stoff wird durch Auflösen in Methaol, Behandlung der Lösung mit Aktivkohle und Abdampfen des Lösungsmittels gereinigt, wobei man 19,1 g weisse Kristalle vom Schmelzpunkt 150—1°C erhält. Diese Kristalle besitzen die Elementaranalyse C 27,90%, H 7,63%, N 16,80% und P 18,24%. $^{1}H$— und $^{13}C$—NMR zeigen, dss diese Kristalle aus

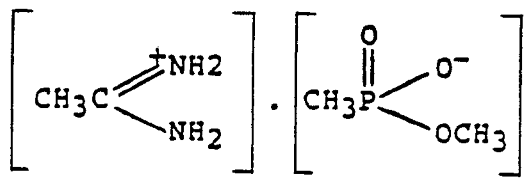

bestehen.

Beispiel 12

Man bringt 88 g (1 Mol) N,N'-Dimethylharnstoff und 124 g (1 Mol) Dimethyl-methylphosphonat in einer Stickstoffatmosphäre insgesamt 32 Stunden lang bei 140°C zur Reaktion. Nach Entfernung nicht umgesetzter Ausgangsstoffe bis zu einer Temperatur von 140°C bei 15,6 mbar Druck erhält man dabei 184,4 g farblose Flüssigkeit mit einem Phosphorgehalt von 14,71%. Berechnet für $C_6H_{17}N_2O_4P$, P = 14,62%.

Beispiel 13

Man erhitzt 124 g (1,0 Mol) Dimethyl-methylphosphonat auf 140°C und versetzt im Verlauf von 2 Stunden mit 73 g (0,86 Mol) Pyrrolidon. Das Reaktionsgemisch wird insgesamt 48 Stunden lang auf 140°C erhitzt. Man entfernt nicht umgesetzte Ausgangsstoffe durch Vakuumabstreifung bei 15,6 mbar Druck. Dabei erhält man 113,3 g dunkelgefärbte viskose Flüssigkeit mit einem Phosphorgehalt von 14,57%. Berechnet für $C_7H_{16}NO_4P$, P = 14,83%.

Beispiel 14

Man schmilzt 113 g (1 Mol) Caprolactam in einer Stickstoffatmosphäre durch Erhitzen auf 140°C, versetzt im Verlauf von 2 Stunden mit 124 g (1 Mol) Dimethyl-methylphosphonat und erhitzt das Reaktionsgemisch anschliessend noch weitere 12 Stunden lang auf 140°C und 29 Stunden lang auf 160°C. Nicht umgesetztes Dimethyl-methylphosphonat wird durch Vakuumabstreifung bei 19,6 mbar Druck entfernt. Man löst die so erhaltene dunkelbraune viskose Flüssigkeit in Methanol und entfärbt durch Behandlung mit Aktivkohle. Die schwachgelbe Lösung wird zur Trockne eingedampft und 14 Tage lang bei Raumtemperatur stehen gelassen, währenddessen nicht umgesetztes Caprolactam aus dem Produkt auskristallisiert. Man filtriert zur Entfernung des kristallinen Caprolactams und erhält dabei 154,9 g gelbe viskose Flüssigkeit, die 15 Gew.-% nicht umgesetztes Caprolactam enthält und einen Phosphorgehalt von 9,93% aufweist. Berechnet für $C_9H_{20}NO_4P$, P = 13,08%.

Beispiel 15

Man erhitzt 86 g (1 Mol) Imidazolin und 124 g (1 Mol) Dimethyl-methylphosphonat in einer Stickstoffatmosphäre 24 Stunden lang auf 140°C. Man kühlt auf 60°C, rüstet die Apparatur für Vakuumdestillation um und entfernt nicht umagesetzte Ausgangsstoffe bei 15,6 mbar Druck. Dabei erhält man 169 g schwachbraune viskose Flüssigkeit mit einem Phosporgehalt von 14,62%. Berechnet für $C_6H_{15}N_2O_4P$, P = 14,76%.

Beispiel 16

In einem mit Rührer, Thermometer und über einen Wasserkühler zu einer eisgekühlten Vorlage führender Fraktionierkolonne ausgerüsteten Vierhalskolben erhitzt man 90 g (2 Mol) Formamid und 124 g (1 Mol) Dimethyl-methylphosphonat insgesamt 18 1/2 Stunden lang auf 140°C. Das Methylformiatnebenprodukt wird über die Fraktionierkolonne abgetrennt.

Nach Abkühlung auf 60°C werden nicht umgesetzte Ausgangsstoffe durch Vakuumabstreifung bei 15,6 mbar bis zu einer Endtemperatur von 120°C entfernt. Das dunkel gefärbte Produkt löst man in 200 ml Methanol und behandelt mit Aktivkohle. Nach Entfernung des Methanols erhält man dabei 142,5 g gelb gefärbte viskose Flüssigkeit mit Phosphorgehalt = 19,97%. Berechnet für $C_3H_{11}N_2O_3P$, P = 20,12%.

$^1H$—, $^{31}P$ und $^{13}C$—NMR-Spektroskopie zeigt, dass das Produkt aus

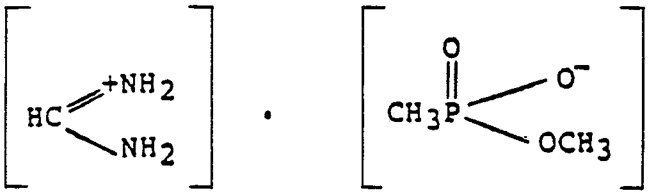

besteht.

Beispiel 17

Man beschickt einen Kolben mit 360 g (6 Mol) Harnstoff und erhitzt auf 150°C. Bei dieser Temperatur gibt man im Verlauf von 7 Stunden eine Lösung von 744 g (6 Mol) Dimethyl-methanphosphonat und 74,4 g (4,13 Mol) Wasser zu dem Harnstoff. Man hält die Reaktionsmasse noch eine Stunde bei 150°C, bevor man sie auf 60°C abkühlt und ablaufen lässt. P—NMRT an dem Produkt zeigt, dass 97,8% des DMMP umgesetzt sind.

Dabei erhält man 969,4 g farblose Flüssigkeit mit einer Viskosität von 31560 cp bei 25°C. Die Elementaranalyse ist ähnlich der in Beispiel 2.

Beispiele 18—35

Die nachfolgenden Beispiele erläutern, wie flammhemmende Polyurethanhartschaumzusammensetzungen leicht aus Polyolen und polymerem Diphenylmethandiisocyanat erfindungsgemäss hergestellt werden können.

Zur Veranschaulichung der Flammschutzwirkung wird die folgende Schaumformulierung verwendet.

| Reaktionspartner | Konzentration (Gewichtsteile) |
|---|---|
| Thanol® R650x[1] | 100 |
| Wasser | 0,2 |
| Silicontensid | 2 |
| Trichlorfluormethan | 40 (für Schaumdichte 30 ± 1 kg/$m^3$) |
| Flammschutzmittel | 10 |
| Suprasec® DND[2] | auf Index 1,05 |

1. Thanol® ist ein aromatisches Polyol.
2. Suprasec® ist ein polymeres Diphenylmethandiisocyanat.

Die obigen Bestandteile werden 10 Sekunden lang in einem Schnellrührer (2000 U.p.M.) bei Raumtemperatur vermischt, wobei man das Isocyanat zuletzt zugibt, und dann sofort in eine Kartonform gegossen. Die einsetzende exotherme Reaktion lässt man zum freien Aufsteigen des Schaums ablaufen. Die Zeit, in welcher der Schaum klebfrei wird, bezeichnet man als die Klebfreizeit. Nach Erreichen der Klebfreizeit wird der Schaum 3 Tage lang bei Raumtemperatur gealtert.

Aus dem Schaum werden Proben geschnitten und der Grenzsauerstoffindexprüfung sowie der senkrechten Brandprüfung B2 nach DIN 4102 unterzogen. Die Ergebnisse sind in der nachfolgenden Tabelle angegeben, im Vergleich mit demselben, ohne Flammschutzmittel hergestellten Schaumstoff. Die gemäss Beispielen 18—35 erhaltenen Schäume zeigen keine Aufspaltung, keinen Verzug und kein Versengen.

| Bsp. | Zusatz | Sauerstoffindex | Prüfung B2 nach DIN 4102 | | | Schaumgrössen* | | |
|---|---|---|---|---|---|---|---|---|
| | | | Zeit bis zur Kennmarke (s) | Maximale Brennhöhe (cm) | Brennzeit (s) | CZ (s) | SZ (s) | KFZ (s) |
| - | ohne | <21 | 3 | >20 | >60 | 17 | 70 | 120 |
| 18 | Produkt aus Beispiel 1 | 23,5 | 5 | 15 | 13 | 19 | 76 | 131 |
| 19 | Produkt aus Beispiel 2 | 25,1 | - | 13 | 13 | 16 | 36 | 64 |
| 20 | Produkt aus Beispiel 3 | 24,9 | 5 | 15 | 9 | 14 | 50 | 87 |
| 21 | Produkt aus Beispiel 4 | 25,6 | - | 14 | 12 | 6 | 32 | 71 |
| 22 | Produkt aus Beispiel 5 | 24,2 | - | 14 | 11 | 23 | 130 | 205 |
| 23 | Produkt aus Beispiel 6 | 23,7 | 5 | 15 | 10 | 10 | 24 | 32 |
| 24 | Produkt aus Beispiel 7 | 24,2 | 5 | 15 | 11 | 15 | 33 | 43 |
| 25 | Produkt aus Beispiel 8 | 25,1 | - | 13 | 10 | 11 | 27 | 37 |
| 26 | Produkt aus Beispiel 9 | 24,3 | 6 | 16 | 9 | 13 | 31 | 46 |
| 27 | Produkt aus Beispiel 10 | 22,4 | 4 | 16 | 14 | 23 | 120 | 150 |
| 28 | Produkt aus Beispiel 11 | 24,5 | - | 14 | 9 | 17 | 54 | 90 |
| 29 | Produkt aus Beispiel 12 | 24,3 | 5 | 16 | 10 | 13 | 31 | 46 |
| 30 | 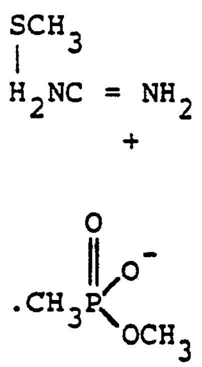 | 24,4 | 5 | 15 | 15 | 20 | 110 | 147 |
| 31 | Gemisch** | 25,4 | - | 12 | 11 | 11 | 25 | 39 |
| 32 | Produkt aus Beispiel 13 | 24,2 | 5 | 15 | 10 | 15 | 32 | 59 |
| 33 | Produkt aus Beispiel 14 | 23,8 | 6 | 16 | 13 | 15 | 37 | 64 |

| Bsp. | Zusatz | Sauerstoffindex | Prüfung B2 nach DIN 4102 | | | Schaumgrössen* | | |
|---|---|---|---|---|---|---|---|---|
| | | | Zeit bis zur Kennmarke (s) | Maximale Kennhöhe (cm) | Brennzeit (s) | CZ (s) | SZ (s) | KFZ (s) |
| 34 | Produkt aus Beispiel 15 | 24,2 | 5 | 15 | 15 | 11 | 30 | 64 |
| 35 | Produkt aus Beispiel 16 | 25,5 | - | 14 | 11 | 6 | 19 | 57 |

*CZ = Cremezeit

SZ = Steigzeit

KFZ = Klebfreizeit

** Das in Beispiel 31 verwendete Gemisch besteht aus 5 Teilen des Produkts aus Beispiel 2 und 19 Teilen eines durch Umsetzung von 49,6 Teilen Dimethyl-methylphosphonat und 245,6 Teilen einer mit Propylenoxyd umgesetzen p-Nonylphenyl/Formaldehyd/Diäthanolamin-Mannichbase (unter dem Markennamen Thanol R650x im Handel erhältlich) unter Rühren bei 120°C in einer Stickstoffatmosphäre erhaltenen Produkts. Nach 4 Stunden Nachrühren bei 120°C überführt man das Gemisch in einen Rotationsverdampfer und entfernt flüchtige Stoffe durch 2 Stunden langes Erhitzen auf 90°C bei 133 Pa Druck.

Beispiel 36—38

Die für Beispiele 18—35 angegebene Formulierung wird ohne Trichlorfluormethan und Suprasec® DND hergestellt und bei einer Temperatur von 50°C gelagert. Nach 1 und 2 Monaten entnimmt man Proben, kühlt auf 23°C und misst ihre Viskosität (Brookfield). Weitere 112,2 Teile werden mit 40 Teilen Trichlorfluormethan verdünnt und dann mit Suprasec DND nach der in Beispielen 18—35 angegebenen Methode auf Index 1,05 vermischt.

Die Ergebnisse sind in der nachfolgenden Tabelle angegeben:

| Bsp. | Zusatz | Lagerzeit bei 50°C | Viskosität % Zunahme | Schaumgrössen | | | Sauerstoffindex % |
|---|---|---|---|---|---|---|---|
| | | | | CZ (s) | SZ (s) | KFZ (s) | |
| 36 | Produkt aus Beispiel 2 | 0 | 0 | 16 | 36 | 64 | 25,2 |
| 37 | " | 1 Monat | 3 | 13 | 32 | 53 | 24,9 |
| 38 | " | 2 Monate | 17 | 13 | 32 | 55 | 25,2 |
| | DMMP | 0 | 0 | 21 | 65 | 130 | 25,4 |
| | " | 1 Monat | 700 | Schaum ungenügender Qualität | | | |
| | " | 2 Monate | 1747 | | | | |

**Patentansprüche**

1. Polyurethane oder Polyisocyanurate mit einer darin eingebauten, flammhemmenden Menge eines Salzs, gebildet durch Umsetzung von Dimethyl-methylphosphonat, Monomethyl-methylphosphonat oder Methylphosphonsäure mit einer Verbindung der allgemeinen Formel (I)

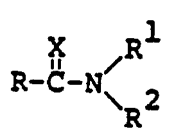

I

worin X für O, S oder NH, $R^1$ für H, Alkyl mit 1 bis 4 Kohlenstoffatomen, Alkenyl mit bis zu 4 Kohlenstoffatomen, CN, $CONH_2$ oder $NH_2$ und $R^2$ für H, Alkyl mit 1 bis 4 Kohlenstoffatomen oder Alkenyl

mit bis zu 4 Kohlenstoffatomen stehen, oder $R^1$ und $R^2$ zusammen mit dem Stickstoffatom, an das sie gebunden sind, einen heterocyclischen Ring mit bis zu 6 Kohlenstoffatomen bilden, der gegebenenfalls ein weiteres Heteroatom enthalten kann, und R für H, eine Alkylgruppe mit 1 bis 8 Kohlenstoffatomen, eine Arylgruppe mit 6 bis 10 Kohlenstoffatomen, eine Cycloalkylgruppe mit 5 bis 12 Kohlenstoffatomen oder eine heterocyclische Gruppe mit bis zu 9 Ringkohlenstoffatomen steht oder zusammen mit $R^1$ eine Alkylenkette mit 3 bis 10 Kohlenstoffatomen bildet, oder R für eine Gruppe $NHR^3$ steht, worin $R^3$ H, Alkyl mit 1 bis 4 Kohlenstoffatomen, Alkenyl mit bis zu 4 Kohlenstoffatomen, CN, $CONH_2$ oder $NH_2$ bedeutet oder zusammen mit $R^1$ eine Alkylenkette mit 2 oder 3 Kohlenstoffatomen bildet, oder R für eine Gruppe

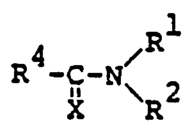

steht, worin $R^1$, $R^2$ und X die obigen Bedeutungen haben und $R^4$ eine direkte Bindung, eine Alkylengruppe mit bis zu 8 Kohlenstoffatomen oder eine Arylengruppe mit 6 bis 10 Kohlenstoffatomen darstellt.

2. Polyurethane oder Polyisocyanurate nach Anspruch 1, dadurch gekennzeichnet, dass die Verbindung der Formel (I) ferner der allgemeinen Formel (II)

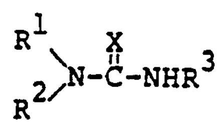

II

entspricht, worin $R^1$, $R^2$, $R^3$ und X die in Anspruch 1 angebenen Bedeutungen haben.

3. Polyurethane oder Polyisocyanurate nach Anspruch 1, dadurch gekennzeichnet, dass die Verbindung der Formel I ferner der allgemeinen Formel (III)

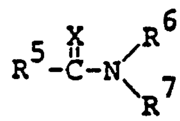

III

entspricht, worin $R^5$ für H, Alkyl mit 1 bis 8 Kohlenstoffatomen oder eine Aryl-, Cycloalkyl- oder heterocyclische Gruppe steht, $R^6$ und $R^7$ gleich oder verschieden sind und H oder eine Alkylgruppe mit 1 bis 4 Kohlenstoffatomen bedeuten und X die in Anspruch 1 angegebene Bedeutung hat.

4. Polyurethane oder Polyisocyanurate nach Anspruch 1, dadurch gekennzeichnet, dass die Verbindung der Formel I ferner der allgemeinen Formel (IV)

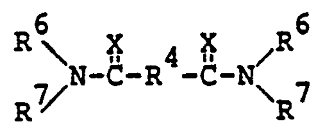

IV

entspricht, worin $R^4$ und X die in Anspruch 1 sowie $R^6$ und $R^7$ die in Anspruch 3 angegebenen Bedeutungen haben.

5. Polyurethane oder Polyisocyanurate nach Anspruch 1, dadurch gekennzeichnet, dass die Verbindung der Formel (I) ferner der allgemeinen Formel (V)

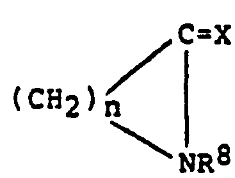

V

entspricht, worin $R^8$ für H oder Alkyl mit 1 bis 4 Kohlenstoffatomen und n für eine ganze Zahl von 3 bis 10 stehen und X die in Anspruch 1 angegebene Bedeutung hat.

6. Polyurethane oder Polyisocyanurate nach Anspruch 1, dadurch gekennzeichnet, dass Verbindung der Formel (I) Harnstoff vorliegt.

7. Zusammensetzungen, dadurch gekennzeichnet, dass sie aus einem Salz nach Anspruch 1 zusammen mit einer weiteren Flammschutzverbindung aus der Gruppe der aliphatischen und aromatischen Bromverbindungen, oxyalkylierten Phosphatestern, Chloralkylphosphaten, -phosphonaten oder Triarylphospnaten bestehen.

8. Zusammensetzungen, dadurch gekennzeichnet, dass sie aus einem Salz nach Anspruch 1 im Gemisch mit Phosphonsäureaminsalzen der allgemeinen Formel (VI) und/oder (VII)

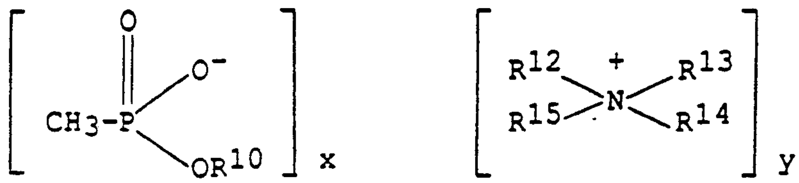

(VI)

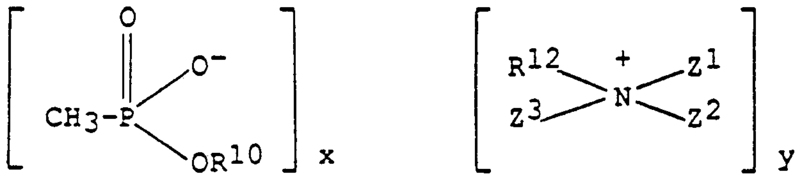

(VII)

bestehen, worin x und y solche ganze Zahlen sind, dass die Anzahl negativer und positiver Ladungen gleich ist, $R^{10}$ und $R^{12}$ für Wasserstoff oder Methyl und $R^{13}$ für eine durch 1—3 gegebenenfalls durch eine Oxyalkylenkette substituierte Hydroxylgruppen substituierte $C_2$—$C_4$-Alkylgruppe stehen, wobei sich jeweils nicht mehr als eine Hydroxylgruppe an einem Kohlenstoffatomen befindet, $R^{14}$ und $R^{15}$ gleich oder verschieden sein und dieselbe Bedeutung wie $R^{13}$ haben oder für Wasserstoff, eine $C_1$—$C_4$-Alkylgruppe oder eine gegebenenfalls durch eine Alkylgruppe mit 1 bis 12 Kohlenstoffatomen, eine gegebenenfalls durch eine Oxyalkylenkette substituierte Hydroxylgruppe und/oder 1—3 Halogenatome am aromatischen Ring substituierte Phenyl- oder Benzylgruppe stehen können oder $R^{15}$ eine Gruppe Formel (VIII)

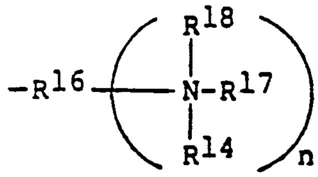

VIII

darstellt, worin $R^{16}$ für eine Alkylengruppe mit 2—4 Kohlenstoffatomen, eine Phenylengruppe, eine Xylylengruppe, eine Diphenylmethangruppe oder eine Gruppe der Formel

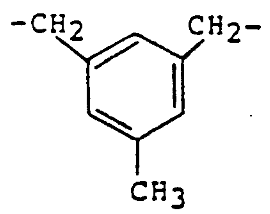

oder

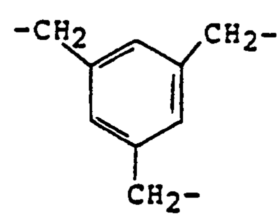

steht, wobei aromatische Ringe in einer Gruppe $R^{16}$ gegebenenfalls durch eine Alkylgruppe mit 1—12 Kohlenstoffatomen, eine gegebenenfalls durch eine Oxyalkylenkette substituierte Hydroxylgruppe und/oder 1—3 Halogenatome substituiert sein können, $R^{17}$ für Wasserstoff oder Methyl, wobei das Stickstoffatom dann eine positive Ladung trägt, steht oder fehlt, $R^{18}$ für Wasserstoff oder eine wie für $R^{13}$ angegebene Gruppe steht und n 1 oder 2 ist, oder $R^{14}$ und $R^{15}$ gegebenenfalls mit dem Stickstoff unter Bildung eines gegebenenfalls ein Sauerstoffatom enthaltenden, 5- oder 6-gliedrigen heterocyclischen Rings verknüpft sind, sowie $Z^1$, $Z^2$ und $Z^3$ gleich oder verschieden sind und Wasserstoff, eine geradkettige oder verzweigte $C_1$—$C_{12}$-Alkyl- oder $C_3$—$C_{12}$-Alkenylgruppe, eine $C_4$—$C_{12}$-Cycloalkylgruppe, eine gegebenenfalls durch eine geradkettige oder verzweigte $C_1$—$C_4$-Alkylgruppe, eine $C_1$—$C_4$-Alkoxygruppe, Amino, Methylamino, Halogen oder Nitro substituierte Phenyl- oder Naphthylgruppe oder eine $C_7$—$C_{12}$-Aralkylgruppe bedeuten.

9. Zusammensetzungen nach Anspruch 7 oder 8, dadurch gekennzeichnet, dass das Verhältnis des Salzes zu der anderen Flammschutzverbindung 5:95 bis 95:5 beträgt.

10. Lagerfähige Zusammensetzungen, dadurch gekennzeichnet, dass sie aus einem zur Reaktion mit einem Polyisocyanat unter Bildung eines Polyurethans befähigten Polyol zusammen mit einem Salz nach Anspruch 1 oder einer Zusammensetzung nach Anspruch 7 oder 8 in Mengen von 1 bis 100 Teilen auf 100 Gewichtsteile Polyol bestehen.

11. Zusammensetzungen nach Anspruch 10, dadurch gekennzeichnet, dass sie 3 bis 50 Teile dieses Salzes oder dieser Zusammensetzung auf 100 Gewichtsteile Polyol enthalten.

12. Verfahren zur Herstellung von Polyurethanen oder Polyisocyanuraten nach Anspruch 1 durch

Umsetzung eines Polyols mit einem Polyisocyanat gegebenenfalls in Gegenwart eines Treibmittels, dadurch gekennzeichnet, dass man einem Reaktionspartner vor der Umsetzung oder dem Reaktionsgemisch ein Salz nach Anspruch 1 oder eine Zusammensetzung nach Anspruch 7 oder 8 zusetzt.

13. Verfahren nach Anspruch 12, dadurch gekennzeichnet, dass die Menge dieses Salzes oder dieser Zusammensetzung 1 bis 100 Gewichtsteile auf 100 Gewichtsteile Polyol beträgt.

14. Verfahren nach Anspruch 12, dadurch gekennzeichnet, dass die Menge dieses Salzes oder dieser Zusammensetzung 3 bis 50 Gewichtsteile auf 100 Gewichtsteile Polyol beträgt.

15. Verfahren nach Anspruch 12, dadurch gekennzeichnet, dass man ein Polyisocyanat mit einer lagerfähigen Zusammensetzung nach Anspruch 10 umsetzt.

16. Salze, gebildet durch Umsetzung von Dimethyl-methylphosphonat, Monomethyl-methylphosphonat oder Methylphosphonsäure mit einer Verbindung der allgemeinen Formel (I) nach Anspruch 1, mit der Massgabe, dass das Salz nicht durch Umsetzung von Methylphosphonsäure mit Dicyandiamid oder Methylphosphonsäure mit Guanidin gebildet wurde.

17. Salze nach Anspruch 16, dadurch gekennzeichnet, dass als Verbindung der Formel (I) Harnstoff vorliegt.

18. Verfahren zur Herstellung von Salzen nach Anspruch 16, dadurch gekennzeichnet, dass man Dimethyl-methylphosphonat, Monomethyl-methylphosphonat oder Methylphosphonsäure mit einer Verbindung der allgemeinen Formel (I) nach Anspruch 1 vermischt, gegebenenfalls in einem wässrigen oder organischen Lösungsmittel und gegebenenfalls in einer Inertgasatmosphäre sowie nötigenfalls unter Erhitzen zwecks Salzbildung.

19. Verfahren nach Anspruch 18, dadurch gekennzeichnet, dass man Dimethyl-methylphosphonat verwendet und die Umsetzung durch Erhitzen auf Temperaturen bis zu 180°C in einer Inertgasatmosphäre durchführt.

**Revendications**

1. Polyuréthannes ou poly-isocyanurates contenant, incorporée en eux, une quantité ignifugeante d'un sel formé par réaction du méthane-phosphonate de diméthyle, du méthane-phosphonate de monométhyle ou de l'acide méthane-phosphonique avec un composé répondant à la formule générale I:

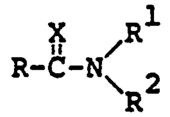

I

dans laquelle

X représente O, S ou NH,

$R^1$ représente H, un alkyle contenant de 1 à 4 atomes de carbone, un alcényle contenant au plus 4 atomes de carbone, CN, $CONH_2$ ou $NH_2$,

$R^2$ représente H, un alkyle contenant de 1 à 4 aomes de carbone ou un alcényle contenant au plus 4 atomes de carbone, ou encore

$R^1$ et $R^2$ forment ensemble, et avec l'atome d'azote auquel ils sont liés, un radical hétérocyclique contenant au plus 6 atomes de carbone, lequel peut éventuellement contenir un hétéroatome supplémentaire, et

R représente H, un alkyle contenant de 1 à 8 atomes de carbone, un aryle contenant de 6 à 10 atomes de carbone, un cycloalkyle contenant de 5 à 12 atomes de carbone ou un radical hétérocyclique contenant au plus 9 atomes de carbone dans son cycle, ou encore R forme avec $R^1$ une chaîne alkylène contenant de 3 à 10 atomes de carbone, ou R représente un radical $NHR^3$ dont le symbole $R^3$ représente l'hydrogène, un alkyle contenant de 1 à 4 atomes de carbone, un alcényle contenant au plus 4 atomes de carbone, CN, $CONH_2$ ou $NH_2$ ou forme, avec $R^1$, une chaîne alkylène contenant 2 ou 3 atomes de carbone, ou R représente un radical:

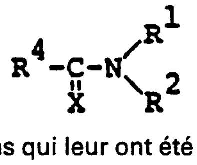

dans lequel $R^1$, $R^2$ et X ont les significations qui leur ont été données plus haut et $R^4$ représente une liaison directe, un radical alkylène contenant au plus 8 atomes de carbone ou un radical arylène contenant de 6 à 10 atomes de carbone.

2. Polyuréthannes ou poly-isocyanurates selon la revendication 1 caractérisés en ce que le composé de formule I répond en outre à la formule générale II

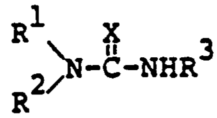

II

dans laquelle $R^1$, $R^2$, $R^3$ et X ont les significations qui leur ont été données à la revendication 1.

3. Polyuréthannes ou poly-isocyanurates selon la revendication 1 caractérisés en ce que le composé de formule I répond en outre à la formule générale III.

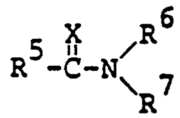

III

dans laquelle $R^5$ repręsente H, un alkyle contenant de 1 à 8 atomes de carbone, un aryle, un cycloalkyle ou un radical hétérocyclique, $R^6$ et $R^7$ représentent chacun, indépendamment l'un de l'autre, un atome d'hydrogène ou un alkyle contenant de 1 à 4 atomes de carbone, et X a la signification qui lui a été donnée à la revendication 1.

4. Polyuréthannes ou poly-isocyanurates selon la revendication 1 caractérisés en ce que le composé de formule I répond en outre à la formule générale IV.

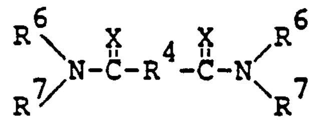

IV

dans laquelle $R^4$ et X ont les significations qui leur ont été données à la revendication 1 tandis que $R^6$ et $R^7$ ont les significations qui leur ont été données à la revendication 3.

5. Polyuréthannes ou poly-isocyanurates selon la revendication 1 caractérisés en ce que le composé de formule I répond en outre à la formule générale V:

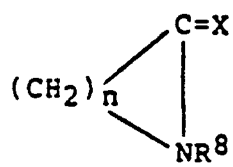

V

dans laquelle $R^8$ represente H ou un alkyle contenant de 1 à 4 atomes de carbone, n désigne un nombre entier de 3 à 10 et X a la signification qui lui a été donnée à la revendication 1.

6. Polyuréthannes et poly-isocyanurates selon la revendication 1 caractérisés en ce qu'ils contiennent de l'urée comme composé de formule I.

7. Compositions caractérisées en ce qu'elles contiennent un sel selon la revendication 1 associé à un autre composé ignifugeant pris dans l'ensemble constitué par le composés bromés aliphatiques ou aromatiques, les esters phosphoriques alcoxylés, les phosphates de chloralkyles, les phosphonates de chloralkyles et les phosphates de triaryles.

8. Compositions caractérisées en ce qu'elles contiennent un sel selon la revendication 1 en mélange avec des sels dérivant d'acides phosphoniques et d'amines qui répondent aux formules générales VI et/ou VII

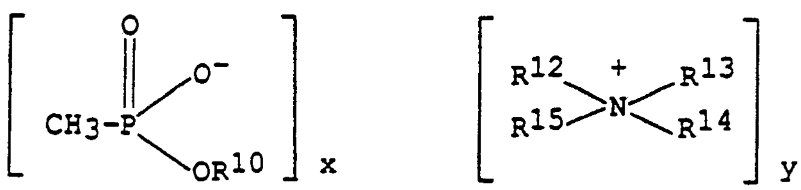

(VI)

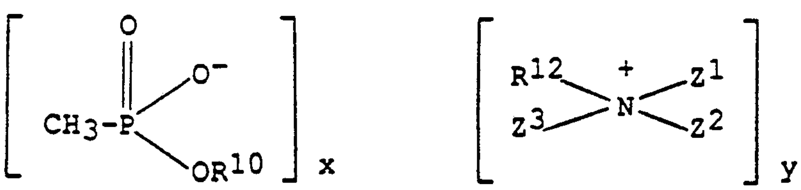

(VII)

dans lesquelles

x et y représentent des nombres entiers tels que le nombre des charges négatives et le nombre des charges positives soient égaux,

$R^{10}$ et $R^{12}$ représentent chacun l'hydrogène ou un méthyle,

$R^{13}$ représente un radical alkyle en $C_2$—$C_4$ qui porte de 1 à 3 radicaux hydroxy éventuellement porteurs d'une chaîne oxyalkylène, avec la condition qu'il n'y ait pas plus d'un radical hydroxy sur un même atome de carbone,

$R^{14}$ et $R^{15}$ représentent chacun, indépendamment l'un de l'autre, un radical pris parmi ceux qui sont définis puor $R^{13}$ ou encore l'hydrogène, un alkyle en $C_1$—$C_4$, ou un radical phényle ou benzyle qui porte éventuellement, sur le noyau aromatique, un alkyle en $C_1$—$C_{12}$, un hydroxy éventuellement porteur d'une chaîne oxy-alkylène et/ou 1, 2 ou 3 atomes d'halogènes, ou

$R^{15}$ représente un radical répondant à formule VIII

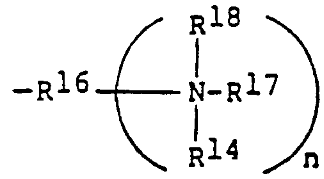

VIII

dans laquelle $R^{16}$ représente un alkylène contenant de 2 à 4 atomes de carbone, un phénylène, un xylylène, un radical de diphénylméthane ou un radical répondant à l'une des formules:

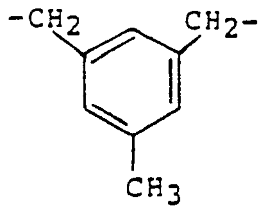

et

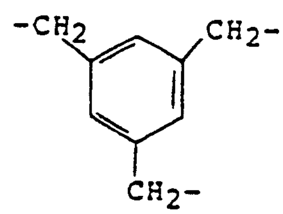

(les noyaux aromatiques présents dans un radical $R^{16}$ pouvant éventuellement porter un alkyle en $C_1$—$C_{12}$, un hydroxy éventuellement porteur d'une chaîne oxy-alkylène et/ou 1, 2 ou 3 atomes d'halogènes), $R^{17}$ représente l'hydrogène ou un méthyle, auquel cas l'atome d'azote porte une charge positive, ou encore $R^{17}$ n'existe pas, $R^{18}$ représente l'hydrogène ou l'un des radicaux indiqués pour $R^{13}$, et n est égal à 1 ou à 2, ou $R^{14}$ et $R^{15}$ sont éventuellement liés l'un à l'autre et forment ainsi, avec l'atome d'azote, un hétérocycle à 5 ou 6 maillons qui contient éventuellement un atome d'oxygène,

$Z^1$, $Z^2$ et $Z^3$ représentent chacun, indépendamment les uns des autres, l'hydrogène, un alkyle en $C_1$—$C_{12}$ linéaire ou ramifié, un alcynyle en $C_3$—$C_{12}$ linéaire ou ramifié, un cycloalkyle en $C_4$—$C_{12}$, un radical phényle ou naphtyle éventuellement porteur d'un alkyle $C_1$—$C_4$ linéaire ou ramifié, d'un alcoxy en $C_1$—$C_4$, d'un amino, d'un méthylamino, d'un halogène ou d'un nitro, ou un aralkyle en $C_7$—$C_{12}$.

9. Compositions selon l'une des revendications 7 et 8, caractérisées en ce que le rapport entre le sel et l'autre composé ignifugeant est compris entre 5:95 et 95:5.

10. Compositions stables au stockage caractéristées en ce qu'elles sont constituées d'un polyol capable de réagir avec un poly-isocyanate en formant un polyuréthanne, et d'un sel selon la revendication 1 ou d'une composition selon l'une des revendications 7 et 8, en des quantités de 1 à 100 parties pour 100 parties en poids du polyol.

11. Compositions selon la revendication 10 caractérisées en ce qu'elles contiennent de 3 à 50 parties dudit sel ou de ladite composition pour 100 parties en poids du polyol.

12. Procédé de préparation de polyuréthannes ou de polyisocyanurates selon la revendication 1 par réaction d'un polyol avec un poly-isocyanate, éventuellement en présence d'un porogène, procédé caractérisé en ce que l'on ajoute à un partenaire réactionnel avant la réaction, ou au mélange réactionnel, un sel selon la revendication 1 ou une composition selon l'une des revendications 7 et 8.

13. Procédé selon la revendication 12 caractérisé en ce que la quantité dudit sel ou de ladite composition est comprise entre 1 et 100 parties en poids pour 100 parties en poids du polyol.

14. Procédé selon la revendication 12 caractérisé en ce que la quantité dudit sel ou de ladite composition est comprise entre 3 et 50 parties en poids pour 100 parties en poids du polyol.

15. Procédé selon la revendication 12 caractérisé en ce que l'on fait réagir un poly-isocyanate avec une composition stable au stockage selon la revendication 10.

16. Sels formés par réaction du méthane-phosphonate de diméthyle, du méthane-phosphonate de monométhyle ou de l'acide méthane-phosphonique avec un composé de formule générale I selon la revendication 1, avec la condition que le sel n'ait pas été formé par réaction de l'acide méthane-phosphonique avec la cyano-guanidine ou de l'acide méthane-phosphonique avec la guanidine.

17. Sels selon la revendication 16 caractérisés en ce qu'ils contiennent de l'urée comme composé de formule I.

18. Procédé pour préparer des sels selon la revendication 16 caractérisé en ce que l'on mélange du méthane-phosphonate de diméthyle, du méthane-phosphonate de monométhyle ou de l'acide méthane-phosphonique avec un composé de formule générale I selon la revendication 1, éventuellement dans un solvant aqueux ou organique, éventuellement dans une atmosphère de gaz inerte et, si besoin est, tout en chauffant, pour former le sel.

19. Procédé selon la revendication 18 caractérisé en ce que l'on utilise le méthane-phosphonate de diméthyle et en ce que l'on effectue la réaction par chauffage à une température pouvant aller jusqu'à 180°C, dans une atmosphère de gaz inerte.

## Claims

1. A polyurethane or polyisocyanurate having incorporated therein a flame retardant amount of a salt formed by reaction between dimethyl methyl phosphonate, monomethyl methyl phosphonate or methyl phosphonic acid, and a compound of the general formula (I)

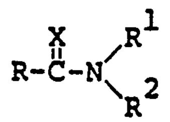

I

in which X is O, S or NH, $R^1$ is H, alkyl of 1 to 4 carbon atoms, alkenyl of up to 4 carbon atoms, CN, $CONH_2$ or $NH_2$, and $R^2$ is H, alkyl of 1 to 4 carbon atoms or alkenyl of up to 4 carbon atoms or $R^1$ and $R^2$ together with the nitrogen atom to which they are attached form a heterocyclic ring of up to 6 carbon atoms which may optionally contain another hetero atom and R is H, an alkyl group of 1 to 8 carbon atoms, an aryl group of 6 to 10 carbon atoms, a cycloalkyl group of 5 to 12 carbon atoms or a heterocyclic group of up to 9 ring carbon atoms, or, together with $R^1$ forms an alkylene chain of 3 to 10 carbon atoms, or R is a group $NHR^3$ in which $R^3$ is H, alkyl of 1 to 4 carbon atoms, alkenyl of up to 4 carbon atoms, CN, $CONH_2$ or $NH_2$ or together with $R^1$ forms an alkylene chain of 2 or 3 carbon atoms, or R is a group

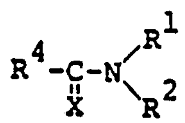

in which $R^1$, $R^2$ and X are as defined above and $R^4$ is a direct bond or an alkylene group of up to 8 carbon atoms or is an arylene group of 6 to 10 carbon atoms.

2. A polyurethane or polyisocyanurate according to claim 1, wherein the compound of the formula (I) is also of the general formula (II)

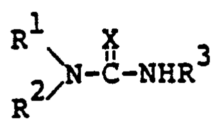

II

in which $R^1$, $R^2$, $R^3$ and X are as defined in claim 1.

3. A polyurethane or polyisocyanurate according to claim 1, wherein the compound of the formula I is also of the general formula (III)

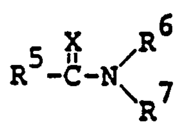

III

in which $R^5$ is H, alkyl of 1 to 8 carbon atoms, or an aryl, cycloalkyl or heterocyclic group, $R^6$ and $R^7$ are the same or different and are H or an alkyl group of 1 to 4 carbon atoms, and X is as defined in claim 1.

4. A polyurethane or polyisocyanurate according to claim 1, wherein the compound of the formula I is also of the general formula (IV)

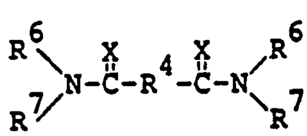

IV

in which $R^4$ and X are as defined in claim 1 and $R^6$ and $R^7$ are as defined in claim 3.

5. A polyurethane or polyisocyanurate according to claim 1, wherein the compound of the formula (I) is also of the general formula (V)

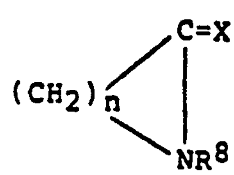

V

in which $R^8$ is H or alkyl of 1 to 4 carbon atoms, and n is an integer from 3 to 10, and X is as defined in claim 1.

6. A polyurethane or polyisocyanurate according to claim 1, wherein the compound of the formula (I) is urea.

7. A composition comprising a salt according to claim 1 together with another flame retardant compound selected from the group comprising aliphatic and aromatic bromine compounds, oxyalkylated phosphate esters, chloroalkyl phosphates, phosphonates or triaryl phosphates.

8. A composition comprising a salt according to claim 1 in admixture with amine salts of phosphonic acids of the general formula (VI) and/or (VII)

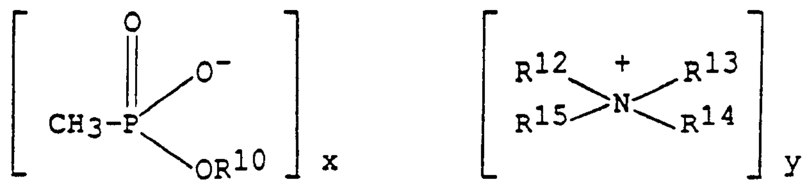

(VI)

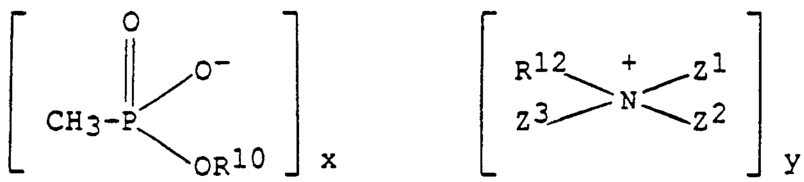

(VII)

in which x and y are integers such that the number of negative and positive charges are the same, $R^{10}$ and $R^{12}$ are hydrogen or methyl and $R^{13}$ is a $C_2$—$C_4$alkyl group substituted by 1—3 hydroxyl groups, which may be substituted by an oxyalkylene chain, there being not more than one hydroxyl group on any one carbon atom, $R^{14}$ and $R^{15}$ may be the same or different and may be a group as defined for $R^{13}$, or hydrogen, a $C_1$—$C_4$-alkyl group, or a phenyl or benzyl group, which may be substituted on the aromatic ring by an alkyl group of 1 to 12 carbon atoms, a hydroxyl group which may be substituted by an oxyalkylene chain, and/or 1—3 halogen atoms, or $R^{15}$ is a group of the formula (VIII)

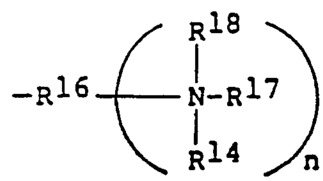

VIII

in which $R^{16}$ is an alkylene group of 2—4 carbon atoms, a phenylene group, a xylylene group, a diphenylmethane group or is a group of the formula

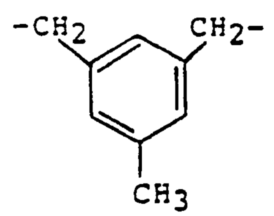

or

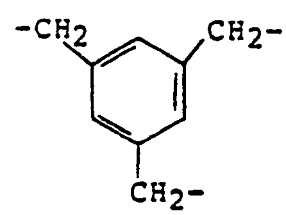

in which aromatic rings in a group $R^{16}$ may be substituted by an alkyl group of 1—12 carbon atoms, a hydroxyl group which may be substituted by an oxyalkylene chain, and/or 1—3 halogen atoms, and $R^{17}$ is hydrogen or methyl, the nitrogen atom then carrying a positive charge, or $R^{17}$ is absent, $R^{18}$ is hydrogen or a group as defined for $R^{13}$ and n is 1 or 2 or $R^{14}$ and $R^{15}$ may be joined to form, with the nitrogen, a 5- or 6-membered heterocyclic ring, optionally containing an oxygen atom, and $Z^1$, $Z^2$ and $Z^3$ are the same or different and are hydrogen, a straight-chain or branched $C_1$—$C_{12}$alkyl or $C_3$—$C_{12}$alkenyl group, a $C_4$—$C_{12}$cycloalkyl group, a phenyl or naphthyl group which may be substituted by a straight-chain or branched $C_1$—$C_4$alkyl group, a $C_1$—$C_4$alkoxy group, amino, methylamino, halogen or nitro, or a $C_7$—$C_{12}$aralkyl group.

9. A composition according to claim 7 or 8, wherein the ratio of the salt to the other flame retardant compound is from 5:95 to 95:5.

10. A storage-stable composition comprising a polyol which is capable of reacting with a polyisocyanate to form a polyurethane, together with a salt according to claim 1 or a composition according to claim 7 or 8 in amounts of from 1 to 100 parts per 100 parts by weight of polyol.

11. A composition according to claim 10 which contains from 3 to 50 parts of said salt or said composition per 100 parts by weight of polyol.

12. A process for preparing a polyurethane or polyisocyanurate according to claim 1 by reacting a

polyol with a polisocyanate, optionally in the presence of a blowing agent which comprises adding a salt according to claim 1 or a composition according to claim 7 or 8 to a reactant before the reaction or to the reaction mixture.

13. A process according to claim 12, wherein the amount of said salt or said composition is from 1 to 100 parts by weight per 100 parts by weight of polyol.

14. A process according to claim 12, wherein the amount of said salt or said composition is from 3 to 50 parts by weight per 100 parts by weight of polyol.

15. A process according to claim 12, which comprises reacting a polyisocyanate with a storage-stable composition according to claim 10.

16. A salt formed by reaction between dimethyl methyl phosphonate, monomethyl methyl phosphonate or methyl phosphonic acid and a compound of the general formula (I) according to claim 1 with the proviso that the salt has not been formed by reaction between methyl phosphonic acid and dicyandiamide or methyl phosphonic acid and guanidine.

17. A salt according to claim 16, wherein the compound of the formula (I) is urea.

18. A process for preparing a salt according to claim 16, which comprises mixing dimethyl methyl phosphonate, monomethyl methyl phosphonate or methyl phosphonic acid with a compound of the general formula (I) according to claim 1, optionally in an aqueous or organic solvent and optionally under an inert gas atmosphere and heating, if necessary, to cause the salt to form.

19. A process according to claim 18, wherein dimethyl methyl phosphonate is used and the reaction is effected by heating at temperatures up to 180°C under an inert gas atmosphere.